# EUROPEAN PATENT SPECIFICATION

(11) **EP 3 915 520 B1**
(45) Date of publication and mention of the grant of the patent: **10.04.2024**
(21) Application number: 20745653.4
(22) Date of filing: 15.01.2020
(51) Int. Cl.: A61F 2/24, A61F 2/95

(54) **IMPLANT LOADING TOOL, COMPRESSION DEVICE AND LOADING SYSTEM**
IMPLANTATLADEWERKZEUG, KOMPRESSIONSVORRICHTUNG UND LADESYSTEM
INSTRUMENT DE CHARGEMENT D'IMPLANT, DISPOSITIF DE COMPRESSION ET SYSTÈME DE CHARGEMENT

(30) Priority: 24.01.2019 CN 201910069078
(43) Date of publication of application: 01.12.2021
(73) Proprietor: Shanghai MicroPort CardioFlow Medtech Co., Ltd., Shanghai 201203 (CN)
(72) Inventor: MEI, Jie, Shanghai 201203 (CN); LIU, Meichen, Shanghai 201203 (CN); WU, Xuwen, Shanghai 201203 (CN); GUI, Baozhu, Shanghai 201203 (CN); CHEN, Guoming, Shanghai 201203 (CN); LI, Yu, Shanghai 201203 (CN)
(74) Representative: Marks & Clerk LLP
(86) International application number: PCT/CN2020/072134
(87) International publication number: WO 2020/151538

(56) References cited:
- WO-A2-2010/121076
- CN-A- 101 460 115
- CN-A- 103 517 689
- CN-A- 104 586 542
- CN-U- 204 814 285
- CN-U- 209 827 106
- US-A1- 2012 330 408
- US-A1- 2012 330 408
- US-A1- 2017 000 634

## Description

### TECHNICAL FIELD

The present invention relates to the technical field of medical instruments and, more specifically, to a loading tool, a compression device and a loading system for an implant.

### BACKGROUND

Interventional aortic valve placement is a brand new minimally invasive valve replacement technology developed internationally in recent years. Its principle is that a valve prosthesis (which includes a stent) is loaded into a delivery device and delivered through a catheter to the aortic root, after the stent is released, it can ensure that the valve prosthesis is fixed to the aortic valve annulus, replacing the degraded original valve, and improving the patient's heart function. This technology can be used to treat aortic valvular disease while the heart is beating without opening the chest, avoiding the huge trauma caused by surgical thoracotomy and cardiac arrest.

This technique requires compressing the stent to achieve a small diameter for loading into the delivery catheter. The stent or the valve on the stent is prone to be damaged due to excessive compression, uneven compression, or local accidental bending, which will eventually cause the function of the stent or valve to be defective or a reduced service life, and may even fail to implant and work abnormally. Especially when the self-expanding stent is loaded, the self-expanding stent is less likely to be fixed and compressed due to its own tension, and it is more likely to be damaged or broken, making it more difficult to load. The technical requirements for the loading personnel are higher, moreover, it also invisibly prolongs the implantation operation time and increases the operation risk.

Generally, when loading the stent with a traditional loading tool, before loading the stent into the delivery catheter, it is necessary to fix the stent to a part in the delivery catheter, such as a fixing element for receiving a lug of the stent, and then gradually complete the loading. Improper fixing of the stent may cause the stent to deform, fail to load, or even damage the stent. Because of the long delivery catheter, the entire delivery device has a large size. In order to ensure that the stent can be loaded into the delivery catheter smoothly, one person is required to operate stent in a fixed position with both hands, and at least one person is additionally required to operate the handle, and hence at least two people or even more people is required to complete the loading process of an entire stent.

Therefore, there is a need for a loading device that has simple operation, high loading efficiency, and can be operated by a single person.

US2017/000634A1 discloses a loading tool.

### SUMMARY OF THE INVENTION

An objective of the present invention is to provide a loading tool, a compression device and a loading system for an implant, so as to solve the problem that the existing loading device needs to be operated by multiple persons when in use.

In order to solve the above-mentioned problems, the present invention provides a loading tool for an implant, according to independent claim 1, configured to cooperate with a compression device to load an implant into a delivery device, the loading tool comprising:
a protective tube, though which the delivery device is able to pass, wherein the protective tube has a distal end and a proximal end from which the implant is able to be inserted in, and the proximal end and the distal end are opposite with each other; and
a protective sleeve, movably sleeved on the protective tube, wherein the protective sleeve has a first connecting portion that is detachably connected with the compression device;
wherein, the protective tube is configured to be connected to the compression device through the first connecting portion, so as to be coaxial with the compression device.

Optionally, the delivery device comprises a capsule, the protective tube is provided with an anti-drop structure, and the anti-drop structure is configured to prevent the capsule from extending beyond the proximal end of the protective tube.

Optionally, the protective tube has an inner wall having a protrusion extending toward an inside of the protective tube, and the protrusion constitutes the anti-drop structure.

Optionally, the proximal end of the protective tube is provided with an anti-dropping fastener having a limiting portion extended to an inside of the protective tube, and the limiting portion constitutes the anti-drop structure.

Optionally, the proximal end of the protective tube has a flared structure having an inner diameter that gradually increases along a direction from the distal end to the proximal end of the protective tube.

According to the invention, a second connecting portion is provided on the protective tube, and the second connecting portion is configured to connect with the protective sleeve to restrict an axial displacement of the protective tube relative to the protective sleeve toward the distal end of the protective tube.

According to the invention, the second connecting portion has a first limiting surface protruding from an outer surface of the protective tube in a circumferential direction thereof; the protective sleeve has a second limiting surface, and the first limiting surface is configured to abut against the second limiting surface to restrict the axial displacement of the protective tube relative to the protective sleeve toward the distal end of the protective tube.

Optionally, the first connecting portion comprises a plurality of engaging teeth which is arranged on an inner wall of the protective sleeve in a circumferential direction thereof and is configured to match engaging grooves of the compression device.

Optionally, the first connecting portion has an internal thread provided on an inner wall of the protective sleeve, and the internal thread is configured to match an external thread of the compression device.

Optionally, an anti-slip structure is provided on an outer wall of the protective sleeve.

In order to solve the above-mentioned problems, the present invention also provides a compression device for implant, configured to cooperate with the above loading tool, the compression device comprising:
a guide cap, having a third connecting portion, wherein the third connecting portion is configured to match and connect with the first connecting portion, so that the protective tube and the guide cap remain coaxial; and
a guide base, detachably connected with the guide cap to compress the implant.

Optionally, the third connecting portion comprises a plurality of engaging grooves which are arranged on an outer wall of the guide cap along a circumferential direction thereof and is configured to match the engaging teeth of the loading tool.

Optionally, the third connecting portion has an external thread provided on an outer wall of the guide cap.

Optionally, the guide cap has a first end and a second end opposite to each other; the guide cap further has a first inner cavity extending through the guide cap, and the first inner cavity has a cross section gradually getting smaller from the first end to the second end; the guide base has a third end and a fourth end opposite to each other; the guide base is configured to enter into the first inner cavity from the first end along a direction from the third end to the fourth end.

In order to solve the above-mentioned problems, the present invention also provides a loading system for implant, comprising:
the above loading tool;
the above compression device; and
a delivery device, the loading tool and the compression device are configured to cooperate with the delivery device to load an implant in the delivery device; wherein, the proximal end of the protective tube is arranged face to face with a second end of the guide cap; the first connecting portion of the protective sleeve is connected to the third connecting portion of the guide cap, so that the protective tube and the guide cap remain coaxial.

Optionally, the implant is a valve stent; the delivery device includes a capsule, and the valve stent is configured to be loaded into the capsule.

In summary, in the loading tool, the compression device and the loading system proposed in the present invention, the loading tool is configured to cooperate with a compression device to load an implant into a delivery device. The loading tool includes a protective tube and a protective sleeve, the protective tube is provided for the delivery device to pass therethrough, and the protective sleeve is detachably connected to the compression device through a first connecting portion, so that the protective tube remains coaxial with the compression device. With such a configuration, no specially-assigned person for fixing the position of the connection between the delivery device and the implant is required, the loading tool and the compression device are fixed stably and firmly, and the implant can be loaded by a single person, with high loading efficiency and simple operation.

### BRIEF DESCRIPTION OF THE DRAWINGS

Those of ordinary skill in the art will understand that the accompanying drawings are provided for a better understanding of the present invention, and do not constitute any limitation on the scope of the present invention.
Fig. 1 is a front view of a protective tube according to Embodiment 1 of the present invention;
Fig. 2 is an axial cross-sectional view of the protective tube shown in Fig. 1;
Fig. 3 is an enlarged view of a part of the protective tube shown in Fig. 2;
Fig. 4 is an axial cross-sectional view of a part of the protective tube according to Embodiment 2 of the present invention;
Fig. 5 is a front view of a protective sleeve according to Embodiment 1 of the present invention;
Fig. 6 is an axial cross-sectional view of the protective sleeve shown in Fig. 5;
Fig. 7 is a front view of a protective sleeve according to Embodiment 2 of the present invention;
Fig. 8 is an axial cross-sectional view of the protective sleeve shown in Fig. 7;
Fig. 9 is a front view of a guide cap according to Embodiment 1 of the present invention;
Fig. 10 is an axial cross-sectional view of the guide cap shown in Fig. 9;
Fig. 11 is a front view of a guide base according to Embodiment 1 of the present invention;
Fig. 12 is an axial cross-sectional view of the guide base shown in Fig. 11;
Fig. 13 is a schematic diagram showing a matching between the guide cap and the guide base provided in Embodiment 1 of the present invention, wherein the fourth connecting portion of the guide cap is connected with the fifth connecting portion of the guide base;
Fig. 14 is an axial cross-sectional view of the guide cap and the guide base shown in Fig. 13;
Fig. 15 is a schematic diagram showing a delivery device according to Embodiment 1 of the present application;
Fig. 16 is a front view (with a partial cross-sectional view) of a loading system provided in Embodiment 1 of the present invention, in which an implant is about to be loaded into a capsule;
Fig. 17 is an axial cross-sectional view of the loading system shown in Fig. 16;
Fig. 18 is an enlarged view of a part of the loading system shown in Fig. 17;
Fig. 19 is an axial cross-sectional view of the loading system provided in Embodiment 1 of the present invention, in which the implant part has been loaded into the capsule;
Fig. 20 is an enlarged view of a part of the loading system shown in Fig. 19;
Fig. 21 is an axial cross-sectional view of the loading system provided in Embodiment 1 of the present invention, in which the protective sleeve and the guide cap have moved to the distal end of the protective tube to expose the proximal end of the protective tube;
Fig. 22 is a schematic diagram of an implant according to Embodiment 1 of the present invention.

In the drawings:
100, protective tube; 101, proximal end; 102, distal end; 110, anti-drop structure; 120, flared structure; 130, first limiting surface; 140, anti-drop fastener;
200, protective sleeve; 210, inner cavity of the protective sleeve; 220, engaging tooth; 230, second limiting surface; 240, anti-slip structure; 250, internal thread;
300, guide cap; 301, first end; 302, second end; 310, first inner cavity; 320, engaging groove; 330, fourth connecting portion; 331, tooth groove;
400, guide base; 401, third end; 402, fourth end; 410, second inner cavity; 420, fixing groove; 430, fifth connecting portion; 440, sixth connecting portion;
5, delivery device; 54, tapered head; 55, fixed head; 56, capsule;
9, valve stent; 91, lug; 92, outflow tract; 93, inflow tract.

### DETAILED DESCRIPTION

To make the objectives, advantages and features of the present invention clearer, the present invention will be further described in detail below with reference to the figures and embodiments. It is noted that the figures are provided in a very simplified form not necessarily presented to scale, with their only intention to facilitate convenience and clarity in explaining the disclosed embodiments. In addition, the structure shown in the drawings is often a part of the actual structure. In particular, the figures generally give emphasis on different details and are accordingly drawn to different scales.

As used herein and in the appended claims, the singular forms of "a," "an", and "the", include plural references unless the context clearly dictates otherwise. As used herein and in the appended claims, the term "or" refers to "and/or" unless the context clearly dictates otherwise.

The invention provides a loading tool, a compression device and a loading system for an implant. The loading tool is used to cooperate with a compression device to load an implant into a delivery device. The loading tool includes a protective tube and a protective sleeve. The protective tube is provided for the delivery device to pass therethrough. The protective tube has a proximal end and a distal end which are opposite with each other. The proximal end is provided for the implant to insert therein, and the distal end is provided for the delivery device to insert therein. The protective sleeve is movably sleeved on the protective tube, and has a first connecting portion detachably connected to the compression device. The protective tube is configured to be connected to the compression device through the first connecting portion, and remains coaxial with the compression device. With such a configuration, no specially-assigned person for fixing the position of the connection between the capsule and the implant is required, the loading tool and the compression device are fixed stably and firmly, and the implant can be loaded by a single person, with high loading efficiency and simple operation.

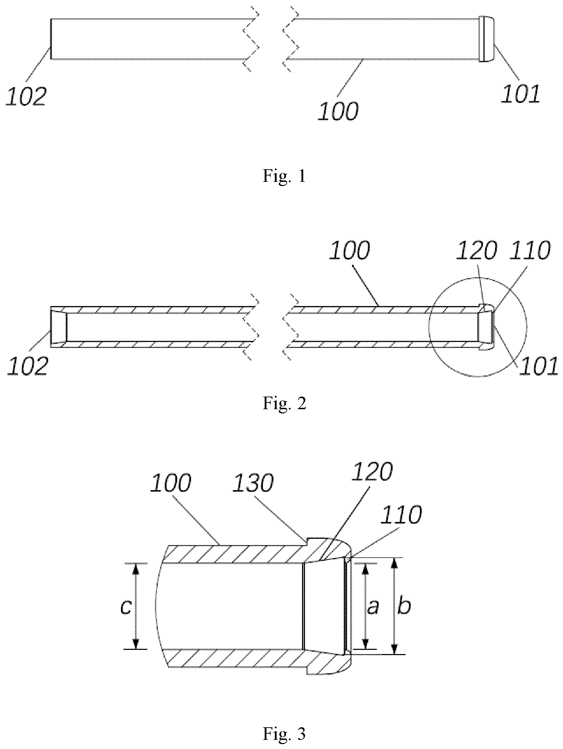

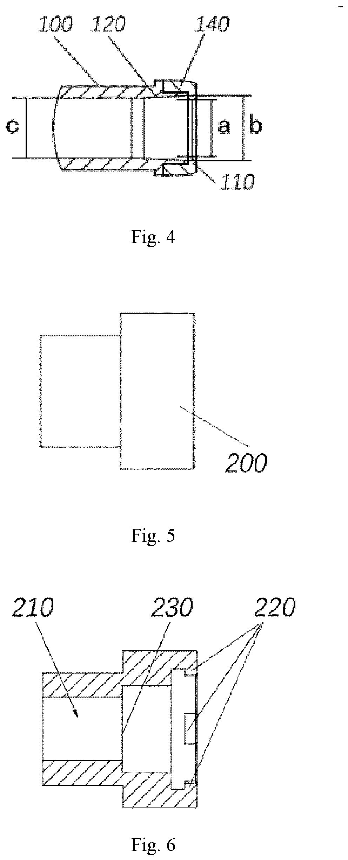

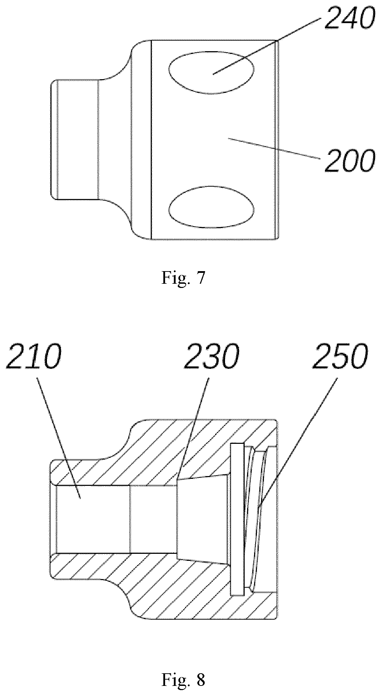

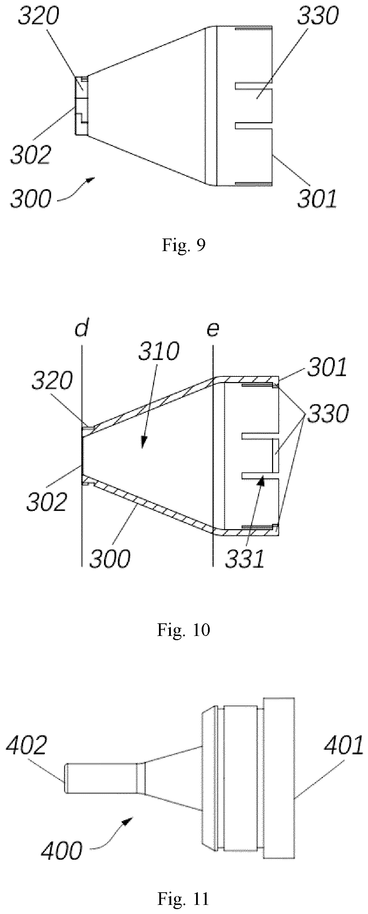

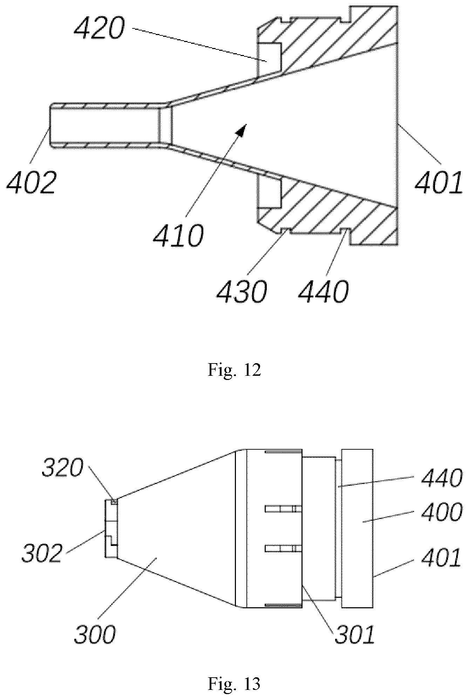

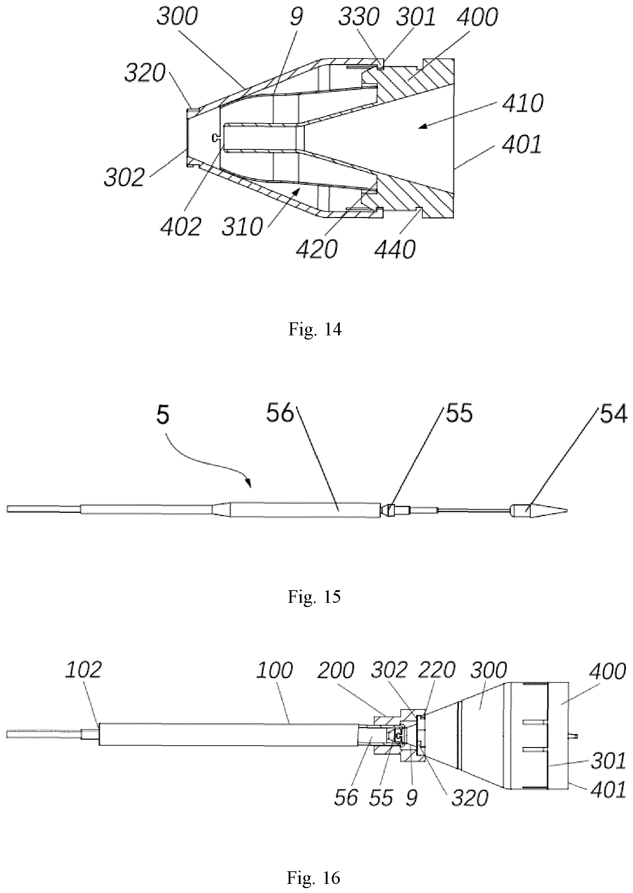

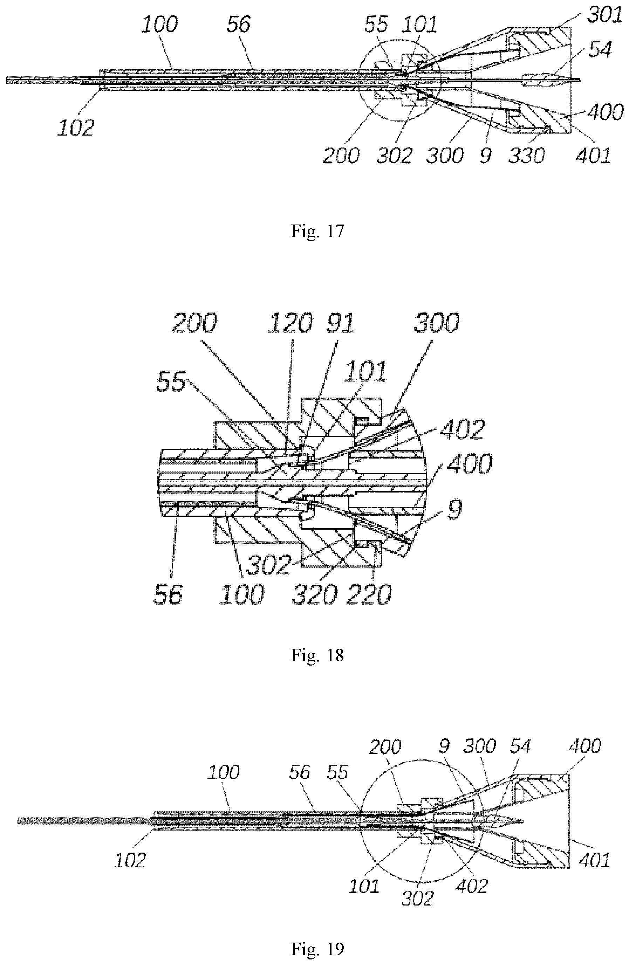

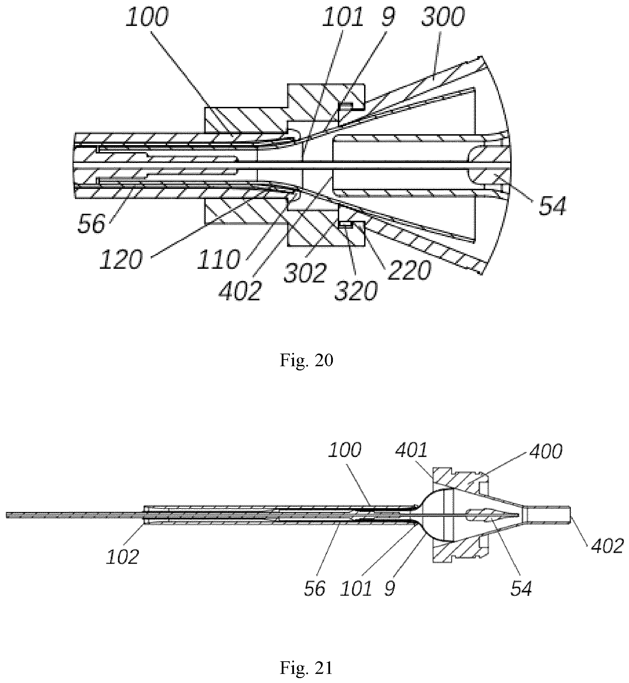

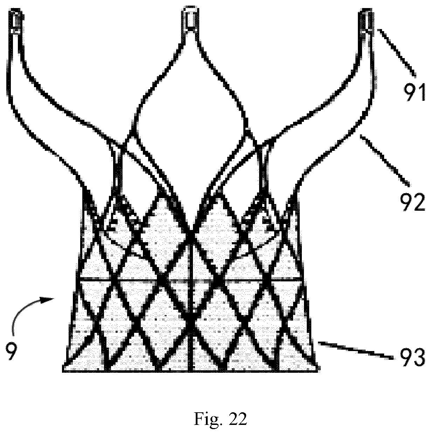

Please refer to Figs. 1 to 22, where Fig. 1 is a front view of the protective tube according to Embodiment 1 of the present invention; Fig. 2 is an axial cross-sectional view of the protective tube shown in Fig. 1; Fig. 3 is an enlarged view of a part of the protective tube shown in Fig. 2; Fig. 4 is an axial cross-sectional view of a part of the protective tube according to Embodiment 2 of the present invention; Fig. 5 is a front view of a protective sleeve according to Embodiment 1 of the present invention; Fig. 6 is an axial cross-sectional view of the protective sleeve shown in Fig. 5; Fig. 7 is a front view of a protective sleeve according to Embodiment 2 of the present invention; Fig. 8 is an axial cross-sectional view of the protective sleeve shown in Fig. 7; Fig. 9 is a front view of a guide cap according to Embodiment 1 of the present invention; Fig. 10 is an axial cross-sectional view of the guide cap shown in Fig. 9; Fig. 11 is a front view of a guide base according to Embodiment 1 of the present invention; Fig. 12 is an axial cross-sectional view of the guide base shown in Fig. 11; Fig. 13 is a schematic diagram showing a matching between the guide cap and the guide base provided in Embodiment 1 of the present invention, wherein the fourth connecting portion of the guide cap is connected with the fifth connecting portion of the guide base; Fig. 14 is an axial cross-sectional view of the guide cap and the guide base shown in Fig. 13; Fig. 15 is a schematic diagram showing a delivery device according to Embodiment 1 of the present application; Fig. 16 is a front view (with a partial cross-sectional view) of a loading system provided in Embodiment 1 of the present invention, in which an implant is about to be loaded into a capsule; Fig. 17 is an axial cross-sectional view of the loading system shown in Fig. 16; Fig. 18 is an enlarged view of a part of the loading system shown in Fig. 17; Fig. 19 is an axial cross-sectional view of the loading system provided in Embodiment 1 of the present invention, in which the implant part has been loaded into the capsule; Fig. 20 is an enlarged view of a part of the loading system shown in Fig. 19; Fig. 21 is an axial cross-sectional view of the loading system provided in Embodiment 1 of the present invention, in which the protective sleeve and the guide cap have moved to the distal end of the protective tube to expose the proximal end of the protective tube; Fig. 22 is a schematic diagram of an implant according to Embodiment 1 of the present invention.

The description is given below with reference to the drawings.

### Embodiment 1

Please refer to Figs 1 to 3 and 5 to 6. This embodiment provides a loading tool for an implant, and the loading tool is configured for cooperating with a compression device to load the implant into a delivery device. The loading tool includes a protective tube 100 and a protective sleeve 200. The protective tube 100 has a proximal end 101 and a distal end 102 which are opposite to each other. Here, the proximal end 101 and the distal end 102 are two opposite ends of the protective tube 100. The protective tube 100 is provided for a delivery device to pass through, and preferably the delivery device includes a capsule that passes through the protective tube 100. The proximal end 101 is provided for an implant to be inserted therein, the distal end 102 is provided for the delivery device to be inserted therein. The protective sleeve 200 is movably sleeved on the protective tube 100. The protective sleeve 200 has a first connecting portion configured to detachably connect with the compression device of the implant. When the first connecting portion is connected with the compression device, the protective sleeve 200 can keep the protective tube 100 coaxial with the compression device.

Further, please refer to Figs. 9 to 14, this embodiment provides a compression device for an implant, and the compression device is used to cooperate with a loading tool for the implant. The compression device includes a guide cap 300 and a guide base 400. The guide cap 300 has a third connecting portion configured to connect with the first connecting portion, so as to keep the protective tube 100 coaxial with the guide base 300. The guide base 400 and the guide cap 300 are detachably connected with each other for compressing the implant.

Furthermore, please refer to Figs. 15 to 21, this embodiment also provides a loading system for an implant. The loading system includes the above-mentioned loading tool and compression device, and also includes a delivery device 5. The delivery device 5 preferably includes a capsule 56. The loading tool and the compression device are used to cooperate with the delivery device 5 to load the implant in the capsule 56 of the delivery device 5. The proximal end 101 of the protective tube 100 and the second end 302 of the guide cap 300 are arranged face to face. The first connecting portion of the protective sleeve 200 is connected to the third connecting portion of the guide cap 300 to keep the protective tube 100 coaxial with the guide cap 300. With this configuration, no specially-assigned person for fixing the position of the connection between the capsule 56 and the implant is required, because the connection between the protective sleeve 200 and the guide cap 300 can ensure that the compression device and the protective tube 100 remain coaxial with each other, ensuring that the implant can be loaded by a single person, with high loading efficiency and simple operation.

In the following, a valve stent 9 is taken as an example of an implant to describe in detail the loading tool, compression device, and loading system provided in this embodiment. As shown in Fig. 22, the valve stent 9 includes an outflow tract 92, an inflow tract 93 and a lug 91. The valve stent 9 is compressed and loaded into the capsule 56 by the loading system provided in this embodiment with the assistance of the delivery device 5. Then in use, the valve stent 9 is delivered in its contracted configuration to a target area in the patient's body through the catheter of the delivery device 5 and released to form an expanded configuration as shown in Fig. 22. It should be understood that the implant here refers to a compressible implant, such as a heart valve stent, but is not limited to the valve stent 9. The present invention does not specifically limit the geometry and material of the implant, which can be any existing implant in the art.

Please refer to Figs. 1 and 2, and in combination with Fig. 15, the protective tube 100 has a hollow inner cavity whose inner diameter matches the outer diameter of the capsule 56, for example, the inner diameter of the protective tube 100 is slightly larger than the outer diameter of the capsule 56, so that the capsule 56 is movably inserted. As shown in Fig. 6, the protective sleeve 200 has an inner cavity 210 whose inner diameter matches the outer diameter of the protective tube 100, for example, the inner diameter of the inner cavity 210 is slightly larger than the outer diameter of the protective tube 100, so that the protective sleeve 200 is movably sleeved on the protective tube 100. Specifically, when the protective sleeve 200 is sleeved on the protective tube 100, the protective sleeve 200 and the protective tube 100 can move axially and remain coaxial with each other. In this way, the radial displacement of the protective sleeve 200 relative to the protective tube 100 can be restricted to ensure that when the protective sleeve 200 is connected to the third connecting portion of the guide cap 300 through the first connecting portion, the protective tube 100 has no radial displacement relative to the guide cap 300.

Preferably, as shown in Figs. 6, 9 and 16, in this embodiment, the first connecting portion of the protective sleeve 200 includes a plurality of engaging teeth 220 circumferentially arranged at the inner wall of the protective sleeve 200. The third connecting portion of the guide cap 300 includes a plurality of engaging grooves 320 circumferentially arranged on the outer wall of the guide cap 300. The number of the engaging teeth 220 is same as that of the engaging groove 320, and the size and position of the engaging teeth 220 are matched with those of the engaging groove 320, so that the engaging teeth 220 and the engaging groove 320 can be engaged with each other to limit the position of the protective sleeve 200 in such a way that there's no axial displacement or radial displacement relative to the guide cap 300, and then the position of the protective tube 100 is limited by the inner cavity 210 of the protective sleeve 200 in such a way that there is at least no radial displacement relative to the guide cap 300, and preferably, for the protective tube 100, there is also no axial displacement relative to the guide cap 300. More preferably, the protective tube 100, the protective sleeve 200 and the guide cap 300 are coaxial with each other when the engaging teeth 220 are engaged with the engaging groove 320.

Further, as shown in Fig. 3, the proximal end 101 of the protective tube 100 is provided with a flared structure 120. The inner diameter of the flared structure 120 gradually increases along a direction from the distal end 102 to the proximal end 101 of the protective tube 100. The maximum inner diameter (b) of the flared structure 120 is at a position thereof close to the proximal end 101. The minimum inner diameter of the flared structure 120 is equal to the inner diameter (c) of the protective tube 100. Please refer to Fig. 20, the valve stent 9 will be compressed from the expanded configuration and loaded into the capsule 56 during the loading process of the valve stent 9, the end (the head end) of the capsule 56 receiving the valve stent 9 will undergo a slight shape change (expansion) under the action of the radial support force applied by the valve stent 9. At this time, the flared structure 120 provides support for the head end of the capsule 56. The flared structure 120 fits well with the head end of the capsule 56 to prevent the head end of the capsule 56 from being over-expanded and damaged. In addition, the flared structure 120 can provide a compressive strain to the valve stent 9 during the compression process of the valve stent 9, which can reduce the abrasion of the valve stent 9. It should be understood that the generatrix of the flared structure 120 is not limited to the straight line in Fig. 3, but may also be a curve, such as a convex curve toward the inside of the protective tube 100, or a combination of a straight line and a curve, which is not limited by the present invention. Preferably, in the axial direction, the length of the flared structure 120 is 10%-30% of the length of the capsule.

Furthermore, the proximal end 101 of the protective tube 100 is provided with an anti-drop structure 110 configured to limit the capsule 56 to not go beyond the proximal end 101 of the protective tube 100 during the process of loading the valve stent 9. Preferably, the inner wall of the protective tube 100 is provided with a protrusion extending toward the inside of the protective tube 100, and the protrusion constitutes the anti-drop structure 110. Preferably, the protrusion has a ring shape and is arranged on an inner wall of the protective tube 100 at the proximal end 101 or close to the proximal end 101. The inner diameter (a) of the protrusion is slightly smaller than the inner diameter (c) of the protective tube 100, is greater than or equal to the outer diameter (x) of the capsule 56 in its normal configuration, and less than or equal to the outer diameter (x') of the end of the capsule 56 in its expanded configuration. The inner diameter (a) which is set to be greater than or equal to the outer diameter (x) of the capsule 56 in its normal configuration ensures that the capsule 56 can pass through the protective tube 100 when the valve stent 9 is not loaded, so as to facilitate the passing of the tapered head through the protective tube 100 because the maximum outer diameter of the tapered head is substantially the same as the outer diameter of the capsule 56. The reason for setting the inner diameter (a) to be less than or equal to the outer diameter (x') of the end of the capsule 56 in its expanded configuration is as follows. In the loading process, due to the radial support force of the valve stent 9, the head end of the capsule 56 will undergo a slight shape change (expansion) under the action of the radial support force applied by the valve stent 9. At this time, the outer diameter (x') of the end of the capsule 56 in its expanded configuration is greater than the outer diameter (x) of the capsule 56 in its normal configuration, and only the inner diameter (a) is set to be less than or equal to the outer diameter (x') of the end of the capsule 56 in its expanded configuration, the capsule 56 can be positionally limited at the distal end of the protrusion. The anti-drop structure 110 is provided for positionally limiting the capsule 56, which has been inserted in from the distal end 102, in the protective tube 100 during the loading process of the valve stent 9, so as to prevent the capsule 56 from extending beyond the proximal end 101 of the protective tube 100 and thus avoid a damage when the valve stent 9 is loaded. Specifically, during the loading process of the valve stent 9, the valve stent 9 and the capsule 56 move toward each other. When the valve stent 9 is loaded into the capsule 56 during the process, the capsule 56 will have a movement tendency opposite to the movement direction of the valve stent 9, and under the blocking of the anti-drop structure 110, it can be ensured that the capsule 56 is limited to the inside of the protective tube 100 in the axial direction. It should be understood that the inner diameter (a) of the protrusion is smaller than the inner diameter (c) of the protective tube 100, which means that the inner diameter of the protrusion is smaller than the inner diameter of a portion of the protective tube 100 that is adjacent to the protrusion. In the case of the flared structure 120 being provided at the proximal end 101, the inner diameter (a) of the protrusion is smaller than the inner diameter (b) of a portion of the flared structure 120 adjacent to the proximal end 101. In this case, the flared structure 120 ensures that the inner diameter of the protrusion is not less than the inner diameter of the protective tube 100, the capsule 56 can be closer to the protective tube 100, and the radial position of the capsule 56 in the protective tube 100 is fixed, which is beneficial to realize that the protective tube 100, the protective sleeve 200 and the compression device are coaxial; preferably, the protrusion of the anti-drop structure 110, the flared structure 120 and the protective tube 100 are integrally formed, which ensures a high structural strength. In some other embodiments, the protrusion may not be a ring shaped structure but a plurality of protruding teeth distributed around the axis of the protective tube 100, which can also provide similar effects.

Please refer to Figs 3 and 6, in combination with Fig. 16, the protective tube 100 has a second connecting portion configured to connect with the protective sleeve 200 to restrict the axial displacement of the protective tube 100 relative to the protective sleeve 200 toward the distal end 102. Preferably, the second connecting portion has a first limiting surface 130 protruding from the outer surface of the protective tube 100 in the circumferential direction. The protective sleeve 200 has a second limiting surface 230 configured to abut against the first limiting surface 130. When the protective sleeve 200 is sleeved on the protective tube 100, the two opposing first limiting surface 130 and the second limiting surface 230 abut against each other to restrict the axial displacement of the protective tube 100 relative to the protective sleeve 200 toward the distal end 102. As shown in Figs. 3, 6 and 16, the protective tube 100 is prevented from moving axially relative to the protective sleeve 200 along a direction from the proximal end 101 to the distal end 102 of the protective tube. In accordance with the assembly relation between the inner cavity 210 of the protective sleeve and the protective tube 100 (that is, the protective sleeve 200 is movably sleeved on the protective tube 100), the protective tube 100 does not have a freedom relative to the protective sleeve 200 in an axial direction (toward the distal end 102) or in a radial direction. The connection between the first connecting portion of the protective sleeve 200 and the third connecting portion of the guide cap 300, it can be ensured that the protective tube 100 and the guide cap 300 remain coaxial, so that the valve stent 9 can be loaded into the capsule 56. Preferably, as shown in Fig. 6, the first connecting portion of the protective sleeve 200 has a cavity with an inner diameter larger than the inner diameter of the inner cavity 210 of the protective sleeve, and the cavity is used for accommodating a section of the second connecting portion of the protective tube 100 raised from the outer peripheral surface thereof. The axial cross-sectional shape of the cavity includes, but is not limited to, a rectangle or a trapezoid. It should be understood that the second connecting portion is not limited to the first limiting surface 130 for connecting with the protective sleeve 200, and the protective tube 100 and the protective sleeve 200 can also be connected detachably by means such as interference fit, threaded fit, or snap fit, as long as the protective tube 100 has no axial (along a direction toward the distal end 102) and radial freedom relative to the protective sleeve 200, according to configurations not covered by the present invention. Preferably, the outer surface of the protective sleeve 200 may be stepped to help the operator to grasp and apply force, but the outer surface of the protective sleeve 200 is not limited to a stepped shape, and may also be a cylindrical shape, a cone shape or even an irregular shape, which is not limited in the present invention.

Please refer to Figs. 9-14, the guide cap 300 has a first end 301 and a second end 302 which are opposite to each other, and the guide cap 300 also has a first inner cavity 310. The guide base 400 has a third end 401 and a fourth end 402 which are opposite to each other. The guide base 400 is configured to enter into the first inner cavity 310 from the first end 301 along the direction from the third end 401 to the fourth end 402 to form an assembled structure for compressing the valve stent 9. Preferably, the cross sections of the first inner cavity 310 are gradually getting small in a direction from the first end 301 to the second end 302. In other words, the cross sections of the first inner cavity 310 show a trend for getting smaller in the direction form the first end 301 to the second end 302, which does not mean any cross-section of the first inner cavity 310 must be larger than the cross-sections of the cross-section on the side closer to the second end 302.

Preferably, as shown in Fig. 10, the first cavity 310 of the guide cap 300 includes, in sequence from the first end 301 to the second end 302, a first area and a second area which are connected. The cross-sections of the first area and the second area are all circular, and the inner diameter of the first area (i.e., the area to the right of the cross-section (e) in Fig. 10) remains unchanged along the axial direction of the guide cap 300. The inner diameter of the second area decreases from a side close to the first end 301 to a side close to the second end 302 (that is, the inner diameter of the cross-section (e) in Fig. 10 is greater than the inner diameter of the cross-section (d)). The generatrix of the second area (in geometric terms, the shape of the curved surface can be regarded as the trajectory of a moving line, and the moving line forming the curved surface is called the generatrix) can be a straight line or a curve, such as a convex curve toward the inside of the guide cap 300 or a concave curve, and can also be a combination of a straight line and a curve. More preferably, a smooth transition is formed between the generatrix of the first area and the generatrix of the second area. In practice, the first area is used to contact, abut or connect with the guide base 400, and the second area is used to compress the valve stent 9. More preferably, the end of the first area close to the first end 301 is provided with a plurality of tooth grooves 331 along the circumferential direction, and an interval is formed between every two adjacent ones of the tooth grooves 331, so that the first area can be easily connected to the guide base 400 by expansion.

Further, the guiding cap 300 has a fourth connecting portion 330. The guide base 400 has a fifth connecting portion 430 and a sixth connecting portion 440, and the fifth connecting portion 430 is closer to the fourth end 402 than the sixth connecting portion 440. The fourth connecting portion 330 is configured to detachably connect with the fifth connecting portion 430 and the sixth connecting portion 440 to restrict axial and radial displacements of the guide cap 300 relative to the guide base 400. Since the fifth connecting portion 430 is closer to the fourth end 402, the connection between the fourth connecting portion 330 and the fifth connecting portion 430 is made prior to the connection between the fourth connecting portion 330 and the sixth connecting portion 440, and the connection between the fourth connecting portion 330 and the sixth connecting portion 440 are made after the fourth connecting portion 330 and the fifth connecting portion 430 are disconnected. When the fourth connecting portion 330 and the fifth connecting portion 430 are connected, there is no radial displacement between the guide cap 300 and the guide base 400, and the axial displacement is also restricted and a coaxial stable state is achieved, which facilitates the insertion of the tapered head 54 of the delivery device 5, facilitates the further compression of the valve stent 9, and also prevents the valve stent 9 from tilting or abrasion during the compression process. In comparison, when the fourth connecting portion 330 is connected to the sixth connecting portion 440, the guiding base 400 is deeper into the first inner cavity 310 of the guiding cap 300. The assembled structure formed in this case completes the preliminary compression of the valve stent 9.

Preferably, the fourth connecting portion 330 includes a plurality of male connectors arranged on an end of the intervals close to the first end 301, and the fifth connecting portion 430 and the sixth connecting portion 440 both serve as female connectors (e.g., an annular groove along the circumference of the guide base 400) mating with the male connectors of the fourth connecting portion 330. The guide cap 300 and the guide base 400 are connected through the engagement between the male connectors and the female connectors, which makes it convenient to assemble and operate, and easy to disassemble, thereby improving the compression efficiency of the valve stent 9. The first area of the guide cap 300 is divided into a plurality of sub-parts in the circumferential direction by a plurality of tooth grooves 331. During the process of pushing the guide base 400 into the first inner cavity 310 of the guide cap 300, the multiple sub-parts thus are easy to be elastically deformed outward along a radial direction of the guide cap 300 under the guidance of the guide base 400 so that the male connector of the fourth connecting portion 330 engages with the female connector of the fifth connecting portion 430 or the sixth connecting portion 440, and thus the guide base 400 is prevented from having a radial displacement relative to the guide cap 300. It is understandable that the number of the tooth grooves 331 can vary according to needs, for example the number may be 2-12. The tooth grooves 331 are preferably evenly distributed on the circumference of the guide cap 300, so that the plurality of intervals are symmetrically distributed on the circumference of the guide cap 300, for example, they are axially symmetrical, centrally symmetrical, or rotationally symmetrical. In addition, each of the intervals can be provided with one of the male connectors of the fourth connecting portion 330. Preferably, only some of the intervals are provided with the male connector, so that both a coaxial fixation and an easy disassembly can be ensured. More preferably, the male connectors are centrally symmetrical on the circumference of the guide cap 300 to ensure that the guide cap 300 and the guide base 400 are uniformly stressed with no tilting when they are connected. In some other embodiments, the connection between the fourth connecting portion 330 and the fifth and sixth connecting portion 430, 440 may also be achieved by friction fixing or interference fit, which is not limited in the present invention.

Please refer to Fig. 12, the guide base 400 has a second inner cavity 410 extending through the guide base 400 in the axial direction, and the cross sections of the second inner cavity 410 are gradually getting small in a direction from the third end 401 to the fourth end 402. In other words, it shows a trend for getting smaller, which does not constitute a limitation. Preferably, the second inner cavity 410 includes, from the third end 401 to the fourth end 402, a third area and a fourth area which are connected. The inner diameter of a portion of the third area close to the third end 401 is larger than the inner diameter of a portion of the third area close to the fourth end 402. The inner diameter of a portion of the fourth area close to the third end 401 is equal to the inner diameter of a portion of the third area close to the fourth end 402, and is not less than the inner diameter of a portion of the fourth area close to the fourth end 402. In practice, the third area is used to compress the implant.

Preferably, the cross-sections of the third area and the fourth area are circular, and the generatrix of the third area and the fourth area are straight lines. In other words, the third area is shaped as a truncated cone having a larger bottom surface facing the third end 401, and the fourth area is shaped as a cylinder or a truncated cone. More preferably, a smooth transition is formed between the generatrix of the third area and the generatrix of the fourth area. The second inner cavity 410 configured in this way is used to compress the valve stent 9 and can achieve better results. Of course, it is also possible that no smooth transition is formed between the generatrix of the third area and the generatrix of the fourth area, but a corner is formed instead between straight lines. In an alternative example, one or two of the generatrix of the third area and the generatrix of the fourth area are curved, which can also achieve better results in actual use, and the present invention does not limit in this regard.

Further, as shown in Fig. 12, the outer wall of the guide base 400 has a fixing groove 420 having an opening along the circumferential direction, and the opening of the fixing groove 420 faces towards the fourth end 402. The fixing groove 420 is configured for receiving the valve stent 9 (mainly the inflow tract 93 of the valve stent 9) in such a way that the valve stent 9 abuts against the fixing groove 420 to restrict the axial displacement of the valve stent 9 toward the third end 401.

The use of the loading system for the implant, as well as the structure and principle of the loading system will be described below.

Please refer to Fig. 15, which shows a delivery device 5 including a tapered head 54, a fixed head 55, a capsule 56, a catheter and a handle (not shown). The tapered head 54 is fixedly connected to the fixed head 55, and the capsule 56 is movably sleeved on the fixed head 55 under the control of the handle. The fixing head 55 is used to connect with the lug 91 of the valve stent 9 and serves as a force applying end to apply force to the valve stent 9.

When the loading system is in use, the valve stent 9 is arranged in such a way that the inflow tract 93 of the valve stent 9 is placed on the fixing groove 420 of the guide base 400, and then the guide cap 300 is pressed along a direction from the outflow tract 92 of the valve stent 9 to the guide base 400 (that is, the guide base 400 enters into the first inner cavity 310) to connect the fourth connecting portion 330 of the guide cap 300 with the fifth connecting portion 430 of the guide base 400, so that the guide cap 300 is positionally fixed relative to the guide base 400 in both axial and radial directions. At this time, since one end of the valve stent 9 is positionally limited by the fixing groove 420, the valve stent 9 is compressed for the first time by the second area of the guided cap 300. At this time, since the fourth connecting portion 330 is connected to the fifth connecting portion 430, the guide cap 300 and the guide base 400 form a first locked state, and the guide cap 300, the guide base 400 and the valve stent 9 are in a coaxial stable state, so that it is convenient for the tapered head 54 to pass through for a further compression of the valve stent, as shown in Figs. 13 and 14.

As shown in Figs. 15 to 18, the fixed head 55 is extended out of the capsule 56 under the operation of the handle. Then the tapered head 54 is inserted into the second inner cavity 410 from the fourth end 402 of the guide base 400 (at this time, the guide cap 300 and the guide base 400 are in the first locked state, the valve stent 9 is arranged between the guide cap 300 and the guide base 400), the lug 91 and the fixing head 55 are roughly aligned, and then the guide cap 300 is continuously pushed towards the guide base 400, so that the fourth connecting portion 330 and the sixth connecting portion 440 are engaged to complete the further compression of the valve stent 9. During this process, part of the outflow tract 92 of the valve stent 9 and the lug 91 are pushed out of the second end 302 of the guide cap 300 to form a fixed connection for the guide cap 300, the guide base 400 and the valve stent 9, so that the lug 91 is easy to be locked in the groove of the fixed head 55.

As shown in Figs. 16 and 18, after it is confirmed that the lug 91 is locked into the groove of the fixed head 55, the first connecting portion of the protective sleeve 200 and the third connecting portion of the guide cap 300 are connected so that the protective tube 100, the protective sleeve 200, and the guide cap 300 and the valve stent 9 are coaxial, which achieves a tight connection between the lug 91 and the groove of the fixed head 55, as well as a stable and reliable fixation, and the operator can free his hands to operate the handle at the other end, so that the operation can be performed by a single person. Under the operation of the handle, the capsule 56 is moved towards the inflow tract 93 (that is, towards the third end 401 of the guide base 400, the right side in the figure); relatively, the fixed head 55 drives the valve stent 9 to move toward the inside of the capsule 56, and the valve stent 9 starts to be loaded into the capsule 56. In this process, the valve stent 9 first contacts the proximal end 101 of the protective tube 100, which prevents the valve stent 9 from directly squeezing and rubbing against the end of the capsule 56. Therefore, the protective tube 100 disperses the force applied by the valve stent 9 to the capsule 56, and provides a protection to the capsule 56. As shown in Figs 19 and 20, as the outflow tract 92 of the valve stent 9 is gradually loaded into the capsule 56, the capsule 56 is pushed towards the guide cap 300 due to the relative movement tendency. When the end of the capsule 56 reaches the anti-drop structure 110 of the protective tube 100, it is blocked by the anti-drop structure 110, so that the capsule 56 will not exceed the protection range of the protective tube 100. In addition, the head end of the capsule 56 close to the guide cap 300 (that is, the end where the valve stent 9 enters) will undergo a slight shape change (expansion) due to the radial support force of the valve stent 9, and then fit well with the flared structure 120 of the protective tube 100 at the proximal end thereof, so that the protective tube 100 can better protect the head end of the capsule 56. When the valve stent 9 is loaded into the capsule 56 to a predetermined distance (which can be set differently according to different implants), as shown in Fig. 20, the first stage of the loading process of the valve stent 9 is completed. After the first stage of the loading process of the valve stent 9 is completed, the guide base 400 and the guide cap 300 are disconnected, and the protective sleeve 200 and the guide cap 300 are both moved toward the distal end 102 of the protective tube 100 until the proximal end 101 of the protective tube 100 is exposed (that is, the valve stent 9 is exposed), in order for loading another part of the valve stent 9. During this process, the protective sleeve 200 and the guide cap 300 can be disconnected from each other, or can also be moved together as a whole.

Please refer to Fig. 21, after completing the first stage of the loading process of the valve stent 9 and removing the protective sleeve 200 and the guide cap 300, and exposing the proximal end 101 of the protective tube 100, the guide base 400 is turned upside down (that is, the third end 401 and the fourth end 402 changes their position), the guide base 400 moves toward the capsule 56 (the left side in the figure) along a direction from the fourth end 402 to the third end 401, and the inflow tract 93 of the valve stent 9 is pushed into the fourth area under the action of the third area of the guide base 400, so that the inflow tract 93 is compressed in such a way that the outer diameter thereof is approximately as large as the inner diameter of the capsule 56. Then, under the continued operation of the handle, the capsule 56 is pushed to move forward, and the valve stent 9 is then pulled to move to the left in the figure, so that the valve stent 9 is completely loaded into the capsule 56 to complete the entire loading process of the valve stent 9.

In summary, after the valve stent 9 is installed in the compression device provided by the present invention, the first connecting portion of the protective sleeve 200 is connected with the third connecting portion of the guide cap 300, so that the protective tube 100, the protective sleeve 200 and the guide cap 300 remain coaxial, which is beneficial for fixing the lug 91 of the valve stent 9 to the fixed head 55, the loading tool and the compression device are fixed stably and firmly, and the operator can free his hands to operate the handle at the other end, and thus the operation only requires a single person. At the same time, the loading efficiency and the loading effect are further improved, and the valve stent 9 is prevented from tilting or breaking during the compression process.

### Embodiment 2

Please refer to Figs. 4, 7 and 8, the loading tool, compression device and loading system for the implant in Embodiment 2 of the present invention are basically the same as those in Embodiment 1. The same parts will not be described again, and only the differences will be described below.

As shown in Fig. 4, in this embodiment, the proximal end 101 of the protective tube 100 is provided with an anti-drop fastener 140 made of a material whose hardness is lower than the hardness of the material of the protective tube 100. For example, silica gel or rubber can be used. The axial section of the anti-drop fastener 140 has an L shape whose one end is sleeved on the outer circumference of the proximal end 101 of the protective tube 100, and the other end extends into the protective tube 100 along the radial direction of the protective tube 100 to form a limiting portion. The limiting portion forms the anti-drop structure 110. Similarly, the limiting portion is preferably ring-shaped, and has an inner diameter (a) smaller than the inner diameter (b) of the proximal end 101 of the protective tube 100 adjacent to it. With this arrangement, since the anti-drop structure 110 is a flexible structure, the valve stent 9 can be better protected when the valve stent 9 passes, so as to prevent the anti-drop structure 110 from damaging the valve stent 9. In addition, the flexible anti-drop structure 110 can also better protect the head end of the capsule 56 to prevent the head end of the capsule 56 from being squeezed and damaged.

As shown in Figs. 7 and 8, in this embodiment, the first connecting portion of the protective sleeve 200 has an internal thread 250 arranged on the inner wall of the protective sleeve 200, and the third connecting portion has an external thread provided on the outer wall of the guide cap 300 for matching with the internal thread 250. After the external thread is matched and connected with the internal thread 250, axial and radial displacements of the protective sleeve 200 relative to the compression device can be restricted. Therefore, the protective tube 100 can be fixed relative to the compression device in both axial and radial directions.

In addition, in this embodiment, an anti-slip structure 240 is provided on the outer wall of the protective sleeve 200 to increase friction to help the operator to grasp. The anti-slip structure 240 may be, for example, an anti-slip groove or an anti-slip protruding tooth. The specific structure of the anti-slip structure 240 is not particularly limited in the present invention, as long as it can facilitate grasping or increasing the friction with the operator.

It should be noted that the different parts between the various embodiments in this specification can also be used in combination with each other, which is not limited in the present invention.

The above description is only for the preferred embodiments of the present invention, and is not intended to limit the scope of the present invention.

## Claims

1. A loading tool for an implant, configured to cooperate with a compression device to load an implant into a delivery device (5), the loading tool comprising:
a protective tube (100), through which the delivery device (5) is able to pass, wherein the protective tube (100) has a distal end (102) and a proximal end (101) from which the implant is able to be inserted in, and the proximal end (101) and the distal end (102) are opposite with each other; and
a protective sleeve (200), movably sleeved on the protective tube (100), wherein the protective sleeve (200) has a first connecting portion that is configured to be detachably connected with the compression device;
wherein the protective tube (100) is configured to be connected to the compression device through the first connecting portion, so as to be coaxial with the compression device;
wherein a second connecting portion is provided on the protective tube (100), and the second connecting portion is configured to connect with the protective sleeve (200) to restrict an axial displacement of the protective tube (100) relative to the protective sleeve (200) toward the distal end (102) of the protective tube (100);
wherein the second connecting portion has a first limiting surface (130) protruding from an outer surface of the protective tube (100) in a circumferential direction thereof; the protective sleeve (200) has a second limiting surface (230), and the first limiting surface (130) is configured to abut against the second limiting surface (230) to restrict the axial displacement of the protective tube (100) relative to the protective sleeve (200) toward the distal end (102) of the protective tube (100).

2. The loading tool for the implant according to claim 1, wherein the delivery device (5) comprises a capsule (56), the protective tube (100) is provided with an anti-drop structure (110), and the anti-drop structure (110) is configured to prevent the capsule (56) from extending beyond the proximal end (101) of the protective tube (100).

3. The loading tool for the implant according to claim 2, wherein the protective tube (100) has an inner wall having a protrusion extending toward an inside of the protective tube (100), and the protrusion constitutes the anti-drop structure (110).

4. The loading tool for the implant according to claim 2, wherein the proximal end (101) of the protective tube (100) is provided with an anti-dropping fastener having a limiting portion extended to an inside of the protective tube (100), and the limiting portion constitutes the anti-drop structure (110).

5. The loading tool for the implant according to claim 1, wherein the proximal end (101) of the protective tube (100) has a flared structure (120) having an inner diameter that gradually increases along a direction from the distal end (102) to the proximal end (101) of the protective tube (100).

6. The loading tool for the implant according to claim 1, wherein the first connecting portion comprises a plurality of engaging teeth which are arranged on an inner wall of the protective sleeve (200) in a circumferential direction thereof and are configured to match engaging grooves (320) of the compression device.

7. The loading tool for the implant according to claim 1, wherein the first connecting portion has an internal thread (250) provided on an inner wall of the protective sleeve (200), and the internal thread (250) is configured to match an external thread of the compression device.

8. The loading tool for the implant according to claim 1, wherein an anti-slip structure (240) is provided on an outer wall of the protective sleeve (200).

9. A loading system for an implant, comprising:
the loading tool according to any one of claims 1-8;
a compression device configured to cooperate with the loading tool; the compression device comprising:
a guide cap (300), having a third connecting portion, wherein the third connecting portion is configured to match and connect with the first connecting portion, so that the protective tube (100) and the guide cap (300) remain coaxial; and
a guide base (400), detachably connected with the guide cap (300) to compress the implant; and
a delivery device (5), the loading tool and the compression device are configured to cooperate with the delivery device (5) to load an implant in the delivery device (5); wherein, the proximal end (101) of the protective tube (100) is arranged face to face with a second end (302) of the guide cap (300); the first connecting portion of the protective sleeve (200) is connected to the third connecting portion of the guide cap (300), so that the protective tube (100) and the guide cap (300) remain coaxial.

10. The loading system for the implant according to claim 9, wherein the implant is a valve stent (9); the delivery device (5) includes a capsule (56), and the valve stent (9) is configured to be loaded into the capsule (56).

11. The loading system for the implant according to claim 9, wherein the third connecting portion comprises a plurality of engaging grooves (320) which are arranged on an outer wall of the guide cap (300) along a circumferential direction thereof and is configured to match the engaging teeth of the loading tool.

12. The loading system for the implant according to claim 11, wherein the third connecting portion has an external thread provided on an outer wall of the guide cap (300).

13. The loading system for the implant according to claim 11, wherein the guide cap (300) has a first end (301) and a second end (302) opposite to each other; the guide cap further has a first inner cavity (310) extending through the guide cap (300), and the first inner cavity (310) has a cross section gradually getting smaller from the first end (301) to the second end (302); the guide base (400) has a third end (401) and a fourth end (402) opposite to each other; the guide base (400) is configured to enter into the first inner cavity (310) from the first end (301) along a direction from the third end (401) to the fourth end (402).

## Patentansprüche

1. Ladewerkzeug für ein Implantat, das dafür konfiguriert ist, mit einer Kompressionsvorrichtung zusammenzuwirken, um ein Implantat in eine Zuführungsvorrichtung (5) zu laden, wobei das Ladewerkzeug Folgendes umfasst:
ein Schutzrohr (100), durch das die Zuführungsvorrichtung (5) hindurchgehen kann, wobei das Schutzrohr (100) ein distales Ende (102) und ein proximales Ende (101) aufweist, in welches das Implantat eingeführt werden kann, und das proximale Ende (101) und das distale Ende (102) einander gegenüberliegen; und
eine Schutzhülse (200), die beweglich auf das Schutzrohr (100) aufgeschoben ist, wobei die Schutzhülse (200) einen ersten Verbindungsabschnitt aufweist, der dafür konfiguriert ist, lösbar mit der Kompressionsvorrichtung verbunden zu werden;
wobei das Schutzrohr (100) dafür konfiguriert ist, durch den ersten Verbindungsabschnitt mit der Kompressionsvorrichtung verbunden zu werden, sodass es koaxial mit der Kompressionsvorrichtung ist;
wobei ein zweiter Verbindungsabschnitt an dem Schutzrohr (100) bereitgestellt ist, und der zweite Verbindungsabschnitt dafür konfiguriert ist, mit der Schutzhülse (200) verbunden zu werden, um eine axiale Verschiebung des Schutzrohrs (100) relativ zu der Schutzhülse (200) in Richtung des distalen Endes (102) des Schutzrohrs (100) einzuschränken;
wobei der zweite Verbindungsabschnitt eine erste Begrenzungsfläche (130) aufweist, die von einer Außenfläche des Schutzrohrs (100) in einer Umfangsrichtung davon vorspringt; die Schutzhülse (200) eine zweite Begrenzungsfläche (230) aufweist, und die erste Begrenzungsfläche (130) dafür konfiguriert ist, an die zweite Begrenzungsfläche (230) anzustoßen, um die axiale Verschiebung des Schutzrohrs (100) relativ zu der Schutzhülse (200) in Richtung des distalen Endes (102) des Schutzrohrs (100) einzuschränken.

2. Ladewerkzeug für das Implantat gemäß Anspruch 1, wobei die Zuführungsvorrichtung (5) eine Kapsel (56) umfasst, das Schutzrohr (100) mit einer Fallschutzstruktur (110) versehen ist, und die Fallschutzstruktur (110) dafür konfiguriert ist, zu verhindern, dass sich die Kapsel (56) über das proximale Ende (101) des Schutzrohrs (100) hinaus erstreckt.

3. Ladewerkzeug für das Implantat nach Anspruch 2, wobei das Schutzrohr (100) eine Innenwand mit einem Vorsprung aufweist, der sich in Richtung einer Innenseite des Schutzrohrs (100) erstreckt, und der Vorsprung die Fallschutzstruktur (110) bildet.

4. Ladewerkzeug für das Implantat gemäß Anspruch 2, wobei das proximale Ende (101) des Schutzrohrs (100) mit einem Fallschutz-Befestigungselement versehen ist, das einen Begrenzungsabschnitt aufweist, der zu einer Innenseite des Schutzrohrs (100) hin erweitert ist, und der Begrenzungsabschnitt die Fallschutzstruktur (110) bildet.

5. Ladewerkzeug für das Implantat nach Anspruch 1, wobei das proximale Ende (101) des Schutzrohrs (100) eine aufgeweitete Struktur (120) mit einem Innendurchmesser aufweist, der entlang einer Richtung vom distalen Ende (102) zum proximalen Ende (101) des Schutzrohrs (100) allmählich zunimmt.

6. Ladewerkzeug für das Implantat nach Anspruch 1, wobei der erste Verbindungsabschnitt eine Vielzahl von Eingriffszähnen umfasst, die an einer Innenwand der Schutzhülse (200) in einer Umfangsrichtung davon angeordnet sind und dafür konfiguriert sind, mit Eingriffsnuten (320) der Kompressionsvorrichtung zusammenzupassen.

7. Ladewerkzeug für das Implantat nach Anspruch 1, wobei der erste Verbindungsabschnitt ein Innengewinde (250) aufweist, das an einer Innenwand der Schutzhülse (200) bereitgestellt ist, und das Innengewinde (250) dafür konfiguriert ist, mit einem Außengewinde der Kompressionsvorrichtung zusammenzupassen.

8. Ladewerkzeug für das Implantat nach Anspruch 1, wobei an einer Außenwand der Schutzhülse (200) eine Antirutschstruktur (240) bereitgestellt ist.

9. Ladesystem für ein Implantat, Folgendes umfassend:
das Ladewerkzeug nach einem der Ansprüche 1 bis 8;
eine Kompressionsvorrichtung, die dafür konfiguriert ist, mit dem Ladewerkzeug zusammenzuwirken; wobei die Kompressionsvorrichtung Folgendes umfasst:
eine Führungskappe (300) mit einem dritten Verbindungsabschnitt, wobei der dritte Verbindungsabschnitt dafür konfiguriert ist, mit dem ersten Verbindungsabschnitt zusammenzupassen und mit diesem verbunden zu werden, sodass das Schutzrohr (100) und die Führungskappe (300) koaxial bleiben; und
eine Führungsbasis (400), die abnehmbar mit der Führungskappe (300) verbunden ist, um das Implantat zusammenzudrücken; und
eine Zuführungsvorrichtung (5), das Ladewerkzeug und die Kompressionsvorrichtung sind dafür konfiguriert, mit der Zuführungsvorrichtung (5) zusammenzuwirken, um ein Implantat in die Zuführungsvorrichtung (5) zu laden; wobei das proximale Ende (101) des Schutzrohrs (100) gegenüberliegend zu einem zweiten Ende (302) der Führungskappe (300) angeordnet ist; der erste Verbindungsabschnitt der Schutzhülse (200) mit dem dritten Verbindungsabschnitt der Führungskappe (300) verbunden ist, sodass das Schutzrohr (100) und die Führungskappe (300) koaxial bleiben.

10. Ladesystem für das Implantat nach Anspruch 9, wobei das Implantat ein Herzklappenstent (9) ist; die Zuführungsvorrichtung (5) eine Kapsel (56) einschließt, und der Herzklappenstent (9) dafür konfiguriert ist, in die Kapsel (56) geladen zu werden.

11. Ladesystem für das Implantat nach Anspruch 9, wobei der dritte Verbindungsabschnitt eine Vielzahl von Eingriffsnuten (320) umfasst, die an einer Außenwand der Führungskappe (300) entlang einer Umfangsrichtung davon angeordnet sind, und dafür konfiguriert ist, mit den Eingriffszähnen des Ladewerkzeugs zusammenzupassen.

12. Ladesystem für das Implantat nach Anspruch 11, wobei der dritte Verbindungsabschnitt ein Außengewinde aufweist, das an einer Außenwand der Führungskappe (300) bereitgestellt ist.

13. Ladesystem für das Implantat nach Anspruch 11, wobei die Führungskappe (300) ein erstes Ende (301) und ein zweites Ende (302) aufweist, die einander gegenüberliegen; die Führungskappe ferner einen ersten inneren Hohlraum (310) aufweist, der sich durch die Führungskappe (300) hindurch erstreckt, und der erste innere Hohlraum (310) einen Querschnitt aufweist, der von dem ersten Ende (301) zu dem zweiten Ende (302) allmählich abnimmt; die Führungsbasis (400) ein drittes Ende (401) und ein viertes Ende (402) aufweist, die einander gegenüberliegen; die Führungsbasis (400) dafür konfiguriert ist, in den ersten inneren Hohlraum (310) vom ersten Ende (301) entlang einer Richtung vom dritten Ende (401) zum vierten Ende (402) einzutreten.

## Revendications

1. Instrument de chargement pour un implant, configuré pour coopérer avec un dispositif de compression pour charger un implant dans un dispositif de délivrance (5), l'instrument de chargement comprenant :
un tube protecteur (100) à travers lequel le dispositif de délivrance (5) peut passer, dans lequel le tube protecteur (100) a une extrémité distale (102) et une extrémité proximale (101) dans laquelle l'implant peut être inséré, et l'extrémité proximale (101) et l'extrémité distale (102) sont opposées l'une à l'autre ; et
un manchon protecteur (200), manchonné de manière mobile sur le tube protecteur (100), dans lequel le manchon protecteur (200) a une première portion de raccordement qui est configurée pour être raccordée de manière amovible avec le dispositif de compression ;
dans lequel le tube protecteur (100) est configuré pour être raccordé au dispositif de compression au moyen de la première portion de raccordement, de manière à être coaxial au dispositif de compression ;
dans lequel une deuxième portion de raccordement est fournie sur le tube protecteur (100), et la deuxième portion de raccordement est configurée pour se raccorder avec le manchon protecteur (200) pour restreindre un déplacement axial du tube protecteur (100) par rapport au manchon protecteur (200) vers l'extrémité distale (102) du tube protecteur (100) ;
dans lequel la deuxième portion de raccordement a une première surface de limitation (130) faisant saillie d'une surface externe du tube protecteur (100) dans un sens circonférentiel de celui-ci ; le manchon protecteur (200) a une deuxième surface de limitation (230), et la première surface de limitation (130) est configurée pour venir en butée contre la deuxième surface de limitation (230) pour restreindre le déplacement axial du tube protecteur (100) par rapport au manchon protecteur (200) vers l'extrémité distale (102) du tube protecteur (100).

2. Instrument de chargement pour l'implant selon la revendication 1, dans lequel le dispositif de délivrance (5) comprend une capsule (56), le tube protecteur (100) est doté d'une structure antichute (110), et la structure antichute (110) est configurée pour empêcher la capsule (56) de s'étendre au-delà de l'extrémité proximale (101) du tube protecteur (100).

3. Instrument de chargement pour l'implant selon la revendication 2, dans lequel le tube protecteur (100) a une paroi interne ayant une saillie s'étendant vers un intérieur du tube protecteur (100), et la saillie constitue la structure antichute (110).

4. Instrument de chargement pour l'implant selon la revendication 2, dans lequel l'extrémité proximale (101) du tube protecteur (100) est dotée d'une attache antichute ayant une portion de limitation étendue vers un intérieur du tube protecteur (100), et la portion de limitation constitue la structure antichute (110).

5. Instrument de chargement pour l'implant selon la revendication 1, dans lequel l'extrémité proximale (101) du tube protecteur (100) a une structure évasée (120) ayant un diamètre interne qui augmente progressivement le long d'un sens de l'extrémité distale (102) à l'extrémité proximale (101) du tube protecteur (100).

6. Instrument de chargement pour l'implant selon la revendication 1, dans lequel la première portion de raccordement comprend une pluralité de dents de mise en prise qui sont agencées sur une paroi interne du manchon protecteur (200) dans un sens circonférentiel de celui-ci et sont configurées pour correspondre à des rainures de mise en prise (320) du dispositif de compression.

7. Instrument de chargement pour l'implant selon la revendication 1, dans lequel la première portion de raccordement comporte un filetage interne (250) fourni sur une paroi interne du manchon protecteur (200), et le filetage interne (250) est configuré pour correspondre à un filetage externe du dispositif de compression.

8. Instrument de chargement pour l'implant selon la revendication 1, dans lequel une structure antiglisse (240) est fournie sur une paroi externe du manchon protecteur (200).

9. Système de chargement pour un implant, comprenant :
l'instrument de chargement selon l'une quelconque des revendications 1 à 8 ;
un dispositif de compression configuré pour coopérer avec l'instrument de chargement ; le dispositif de compression comprenant :
un capuchon de guidage (300), ayant une troisième portion de raccordement, dans lequel la troisième portion de raccordement est configurée pour correspondre et se raccorder à la première portion de raccordement de sorte que le tube protecteur (100) et le capuchon de guidage (300) restent coaxiaux ; et
une base de guidage (400), raccordée de manière amovible au capuchon de guidage (300) pour comprimer l'implant ; et
un dispositif de délivrance (5), l'instrument de chargement et le dispositif de compression sont configurés pour coopérer avec le dispositif de délivrance (5) pour charger un implant dans le dispositif de délivrance (5) ; dans lequel, l'extrémité proximale (101) du tube protecteur (100) est agencée face à face avec une deuxième extrémité (302) du capuchon de guidage (300) ; la première portion de raccordement du manchon protecteur (200) est raccordée à la troisième portion de raccordement du capuchon de guidage (300) de sorte que le tube protecteur (100) et le capuchon de guidage (300) restent coaxiaux.

10. Système de chargement pour l'implant selon la revendication 9, dans lequel l'implant est une endoprothèse valvulaire (9) ; le dispositif de délivrance (5) inclut une capsule (56), et l'endoprothèse valvulaire (9) est configurée pour être chargée dans la capsule (56).

11. Système de chargement pour l'implant selon la revendication 9, dans lequel la troisième portion de raccordement comprend une pluralité de rainures de mise en prise (320) qui sont agencées sur une paroi externe du capuchon de guidage (300) le long d'un sens circonférentiel de celui-ci et est configurée pour correspondre aux dents de mise en prise de l'instrument de chargement.

12. Système de chargement pour l'implant selon la revendication 11, dans lequel la troisième portion de raccordement comporte un filetage externe fourni sur une paroi externe du capuchon de guidage (300).

13. Système de chargement pour l'implant selon la revendication 11, dans lequel le capuchon de guidage (300) a une première extrémité (301) et une deuxième extrémité (302) opposées l'une à l'autre ; le capuchon de guidage a, en outre, une première cavité interne (310) s'étendant à travers le capuchon de guidage (300), et la première cavité interne (310) a une section transversale qui se réduit progressivement de la première extrémité (301) à la deuxième extrémité (302) ; la base de guidage (400) a une troisième extrémité (401) et une quatrième extrémité (402) opposées l'une à l'autre ; la base de guidage (400) est configurée pour entrer dans la première cavité interne (310) depuis la première extrémité (301) le long d'un sens depuis la troisième extrémité (401) à la quatrième extrémité (402).
